(19) **Europäisches Patentamt**

**European Patent Office**

**Office européen des brevets**

(11) **EP 1 683 495 A1**

(12) **EUROPEAN PATENT APPLICATION**

(43) Date of publication:
26.07.2006 Bulletin 2006/30

(51) Int Cl.:
*A61B 17/32* (2006.01)

(21) Application number: 06250250.5

(22) Date of filing: 18.01.2006

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **19.01.2005 US 39145**

(71) Applicant: **Depuy Mitek, Inc.
Norwood, MA 02062 (US)**

(72) Inventors:
• **McRury, Ian D.
Medway
Massachusetts 02053 (US)**

• **Sengun, Mehmet Z.
Framingham
Massachusetts 01702 (US)**
• **Weinert, Christopher
Mansfield
Massachusetts 02048 (US)**

(74) Representative: **Mercer, Christopher Paul et al
Carpmaels & Ransford,
43-45 Bloomsbury Square
London WC1A 2RA (GB)**

(54) **Fluid cutting device and method of use**

(57) A fluid jet cutting system that provides more consistent cutting jet performance is disclosed. The system includes a fluid pump for pressurizing fluid having a fluid inlet and a fluid outlet. The pump delivers pressurized fluid to a delivery tube that is in fluid communication with an end effector. The end effector includes an orifice for delivering pressurized fluid to tissue. The power delivered to the pump is controlled to produce a desired fluid cutting jet.

FIG. 1

**Description**

BACKGROUND OF THE INVENTION

High pressure fluid steams provide one method of cutting tissue during a surgical procedure on, for example, non-living tissue or cadavers, or in vitro. Such "fluid jet cutting" has been used to cut, drill, bore, perforate, strip, delaminate, liquefy, ablate, and/or shape a diverse range of tissues, For example, fluid jets have been used to disintegrate and shape eye lens tissue, remove deposits from a vein or artery, and remove or shape meniscal tissue.

In use, a fluid is pressurized and a thin cutting beam is formed as the fluid stream travels through a tiny jet orifice in a surgical hand piece. A surgeon can vary the fluid pressure and/or the surgical hand piece depending on the type of surgical procedure that is performed. For example, selective variation of the jet stream pressure, (e.g., between 1 and 50,000 p.s.i.), allows the surgeon to cut hard bone, soft bone, cartilage and tissue, to strip away tissue exposing underlying organs or vessels or, simply to wash away blood and debris created by the surgical procedure.

One exemplary fluid cutting jet system is disclosed in U.S. Patent No. 6,216,573 to Moutafis et al. and is hereby incorporated by reference in its entirety. The fluid cutting jet system includes a variable pressure pump for generating a high pressure fluid jet. In use, a user can turn on, increase, and/or decrease the pressure of the pump by adjusting dials or buttons.

Despite the existence of these fluid cutting jet systems, there remains a need for fluid jet cutting system that can provide improved cutting performance. Conventional cutting systems often lack robust controllers that can account for variations within the fluid cutting jet system. Such controllers might only limit the maximum pressure in the interest of safety. For example, a simple ON/OFF controller that deactivates the pump in the event of an overpressure situation.

Other controllers, as disclosed in the above referenced Patent, allow a user to turn on the system and increase or decrease pump pressure. However, this system fails to account for the use of different surgical cutting wands and/or orifice dimensions that can vary within manufacturing tolerances. As a result, the cutting performance (i.e., cutting depth) is not consistent for a given pump pressure setting. The lack of robust controller thus results in inconsistent cutting jets that can vary widely with the characteristics of the cutting jet system.

SUMMARY OF THE INVENTION

Disclosed herein are fluid cutting jet control systems that provide improved cutting performance. In one aspect, the control system achieves improved performance by controlling the jet power of the cutting stream. The cutting stream produced is less sensitive to variations, such as, for example dimensions that fluctuate within manufacturing tolerances and/or with different end effectors.

In one aspect, a fluid cutting jet system includes a fluid pump for pressurizing fluid including a fluid inlet and a fluid outlet. A control console communicates with the pump and is adapted to control the fluid jet power of the fluid cutting stream by manipulating pump settings. For example, the control console can include a processor that receives input from a user and sends an output signal to control the pump. A user can enter a cutting set point, and the control console then sends a signal indicating the proper pump setting.

In another aspect, the control console controls the power level of the pump motor. For example, where the pump motor is an electric motor, the control console can control the electrical power delivered to the pump.

The processor can also receive a feedback signal from a sensor and can vary the output signal in response to the feedback signal. Exemplary sensor(s) can be positioned throughout the system. For example, a sensor(s) can be positioned on/in the end effector to measure flow rate and/or pressure. An alternative or additional sensor can provide data to the control console related to the pump, such as, for example, the power delivered to the pump motor.

In another aspect, a method for controlling the fluid cutting jet is disclosed. The method can be used, for example, in surgical procedures that are not suitable for treatment of a human or animal body by surgery or therapy, and the method will involve no diagnostic method practised on the human or animal body. In one embodiment, the method includes providing a fluid cutting jet system having a fluid pump for pressurizing fluid. The pump includes a pump motor for driving the pump, a control console connected to the pump motor and adapted to vary the pump motor power level, and an end effector including a nozzle for delivering pressurized fluid to tissue. Preferably the tissue is non-living tissue or tissue on a cadaver or a recently dead animal or person, in which case the tissue may still be living tissue. The method may be carried out in vitro. The method further includes the steps of inputting a set point and varying the pump motor power level in response to the set point. In one aspect, the set point is representative of fluid jet power.

The method can include the further steps of providing a feedback signal to the control console from at least one sensor positioned to monitor a system variable. In response to the feedback signal, the control console can vary the pump motor power level.

BRIEF DESCRIPTION OF THE DRAWINGS

The invention can be more fully understood from the following detailed description taken in conjunction with the accompanying drawings, in which:

FIG. 1 is a schematic view of an exemplary embodiment of the fluid jet cutting system;

FIG. 2 is a perspective view of another exemplary embodiment of the fluid jet cutting system;

FIG. 3 illustrates that relationship of jet power and cutting depth for the fluid cutting system;

FIG. 4 illustrates the relationship of pump power and jet power for the fluid jet cutting system;

FIG. 5 compares the variation in performance of a fluid cutting jet produced by controlling pump power and a fluid cutting jet produced by controlling pump speed; and

FIG. 6 provides another graphically view of the data shown in FIG. 5.

DETAILED DESCRIPTION OF THE INVENTION

Disclosed herein is a fluid jet cutting control system that provides improved cutting performance. The system includes a pump which delivers a fluid stream to an end effector. The fluid exits the end effector as a cutting stream that can be used to create surgical incisions. In one aspect, improved system performance is achieved by controlling the jet power of the cutting stream. In a further aspect, pump power is manipulated to control the jet power of the cutting stream.

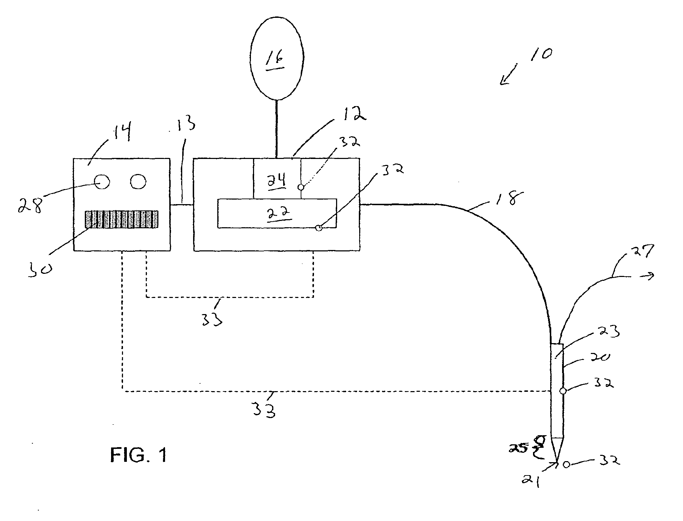

FIG. 1 illustrates an exemplary fluid cutting jet system 10, including a pump 12 that receives fluid from fluid source 16 and delivers the fluid, via a delivery tube 18, to an end effector 20. When the fluid is pressurized by pump 12, it traverses end effector 20 and passes through an orifice 21. Console 14 allows a user to vary the intensity of the fluid stream leaving orifice 21. For example, console 14 allows a surgeon to control the cutting speed of the fluid cutting jet.

Pump 12 can include a pump motor 22 that drives pump assembly 24 to pressurize the fluid. In one aspect, the pump is a positive displacement piston pump, however, one skilled in the art will appreciate that a variety of other pumps are available. For example, other pump mechanisms such as an air intensifier could be substituted.

The delivery tube 18 into which the fluid is delivered is preferably flexible and pressure resistant so that end effector 20 can be moved independently of pump 12. In one embodiment, delivery tube 18 is also adapted to detachably connect to end effector 20 so that different end effectors can be used with system 10. For example, a highpressure quick connect at the end of delivery tube 18 can detachably connect to end effector 20.

End effector 20 can be a handheld or machine controlled surgical jet instrument that is in fluid communication with delivery tube 18. For example, end effector 20 can include a proximal handle portion 23, internal lumen (not shown), and one or more distal jet orifice(s) 21 that are positioned axially or transversely. In addition, end effector 20 can have a tip section 25 that is selectively moldable allowing the user to reshape or bend the jet tip into a desired configuration, or at an angle, thus facilitating positioning at the cutting site. One of skill in the art will appreciate that a variety of jet-creating-end-effector configurations can be used.

End effector 20 can further include a fluid evacuation pathway for the removal of fluid from a surgical site. For example, an exit orifice located near the fluid delivery orifice can receive debris caused by the fluid cutting, as well as, cutting fluid. The removed debris and fluid can travel through exit lumen 27 in fluid contact with the exit orifice. In one aspect, suction may be employed to assist with debris and fluid removal, however, the outflow can also be gravity drained.

The flow of cutting fluid from end effector 20 is preferably controlled by console 14. A user can input a setting into console 14 and the console can output control data. For example, control console 14 can include controls 28 so that a user can vary the system settings, such as, the jet power of the fluid exiting orifice 21. Controls 28 can also be positioned remotely from console 14, such as via a foot switch (not shown in FIG. 1).

Console 14 is preferably in electrical communication with pump 12 to facilitate control of the pump. FIG. 1 illustrates an electrical connection 13 between console 14 and pump 12. One skilled in the art will appreciate that connection 13 can be wireless and/or wired. In addition, while the illustrated console 14 is housed separately from pump 12, console 14 and pump 12 can be disposed within a single body as described below.

In one embodiment, processor 30 controls the pump 12 in response to input from a user and/or a sensor. For example, when user enters a cutting set point, the controller determines the proper pump motor settings and sends an output signal that controls the pump. Processor 30 can include, for example, discrete electronic components, integrated circuit semiconductors, microcontrollers (e.g., stand alone computing units), and combinations thereof. One skilled in the art will appreciate that the variety of known processors can be used to assist with operation of system 10. In addition, where a control system is used, a variety of controllers can be used. For example, the controller can be analog (continuous) or digital (discrete), open or closed loop, and can incorporate single or multiple variables. Exemplary controllers include PI control, PID control, and IMC-based PID control. Simplistic control systems, such as on/off control can also be used. One skilled in the art will appreciate that a variety of other control systems can be implemented in system 10.

Processor 30 can receive signals from sensor(s) located throughout system 10. As shown in FIG. 1, sensors 32 can be positioned in a variety of locations. In one embodiment, sensors 32 are positioned to monitor pump 12. For example, sensors 32 can collect data on pump power, pump speed, pump flow rate, and/or other pump characteristics.

In addition, or as an alternative, system 10 can include other sensors for measuring fluid jet characteristics such as fluid flow rate and/or fluid pressure. In one aspect, sensors are positioned within end effector 20 to measure cutting fluid variables and/or outlet flow rate. For example, sensors can measure flow rate and/or pressure within the internal lumen of end effector 20. Other sensor locations include placement immediately adjacent to fluid jet orifice 21, in console 14, and/or sensors positioned in a surgical field separate from end effector 20.

Sensors 32 are preferably in communication with processor 30 and can transfer data electronically through wires or wirelessly. For example, dotted line 33 illustrates console 14 electronically communicating with sensors 32.

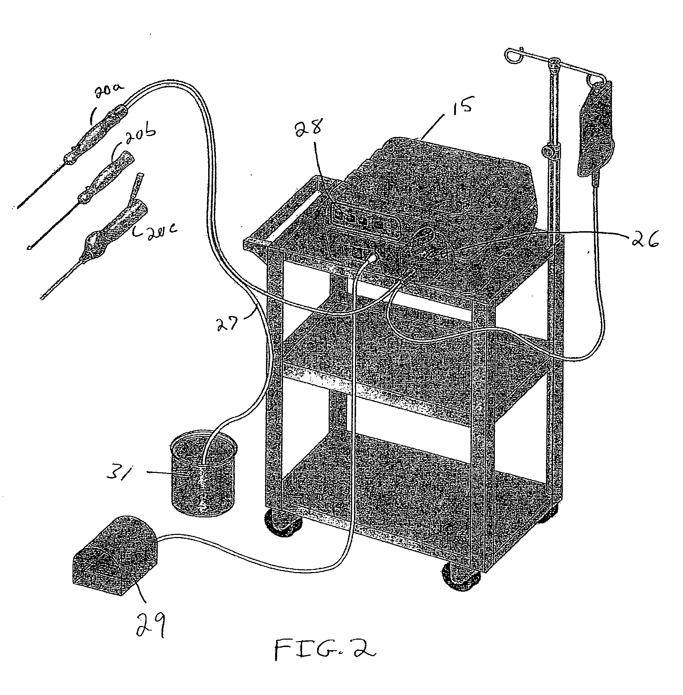

FIG. 2 illustrates another exemplary embodiment of fluid jet cutting system 10 in which control console 14 and at least a portion of the pump (i.e., the pump motor) are housed within the same structure. As shown, housing 15 contains console controls 28 and the pump motor (not shown). The pump may also be controlled by foot switch 29. In use, cutting fluid from fluid source 16 (saline bag) is drawn into the pump and delivered to end effector 20. Effectors 20a, 20b, and 20c can be detachably mated with delivery tube 18. System 10 can also include exit lumen 27 so that fluid and debris can exit to a waste receptacle 31.

To protect against contamination of fluid flowing through the pump and to facilitate sterilization, the pump motor can drive the fluid via a replaceable cartridge 26. In one aspect, cartridge 26 contains the fluid pathway. Pump assembly 24 can act on cartridge 26 and pump the cutting fluid without the pump assembly contacting the fluid. After each use, the cartridge can be replaced.

System 10 can provide consistent cutting jet performance. In one embodiment, the improved performance is obtained by adjusting or controlling system variables to achieve a desired jet power from the fluid cutting stream. The resulting cutting stream can provide consistent cutting depth and is less susceptible to system variations. For example, dimensions that fluctuate within manufacturing tolerances and the substitution of different end effectors have less impact on the cutting performance of system 10. The result is an accurate and precise fluid cutting jet.

Jet power depends on the pressure and volumetric flow rate of the fluid cutting jet. As shown in Equation 1 jet power is the product of pressure and flow rate.

$$\text{Jet Power } = \text{Pressure } x \text{ Volume Flow Rate (Equation 1)}$$

At a constant jet power, a change in one variable (pressure or flow rate) is compensated for by an inverse change in the other variable. For example, if system pressure were to drop, an increase in flow rate of the cutting fluid would compensate.

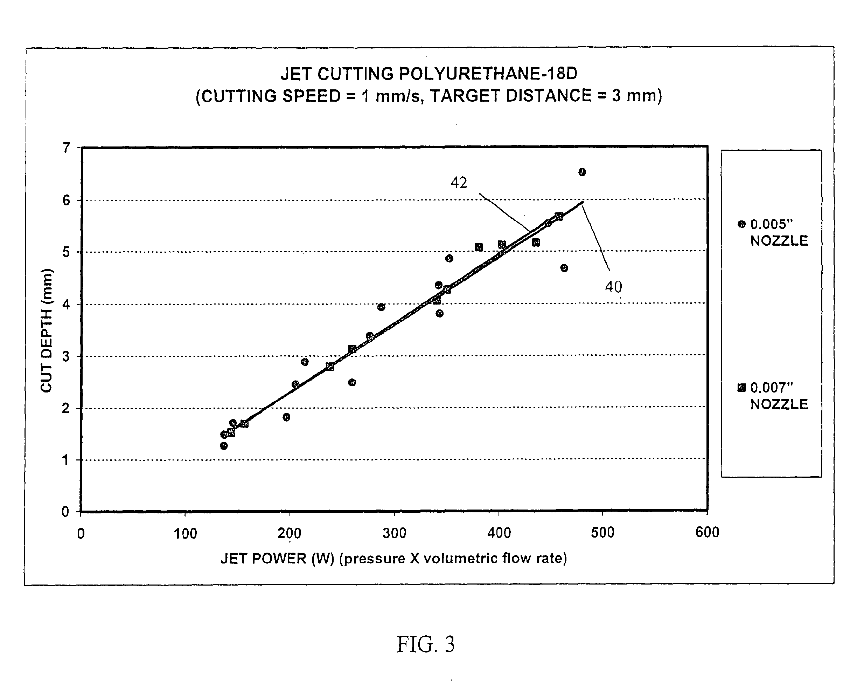

The advantages of controlling jet cutting system 10 based on jet power are illustrated in FIG. 3. As shown, a series of experiments were performed using fluid jet cutting system 10 and monitoring the cutting performance of the fluid stream on polyurethane. The jet power of the fluid stream delivered by the fluid jet cutting system is marked on the x-axis, while the cutting depth of the resulting fluid stream for a given jet power is set forth on the y-axis. The jet power was determined by measuring pressure of the fluid stream and the volume flow rate of the fluid stream. Applicants performed this experiment by measuring the cutting depth of the fluid stream in polyurethane with a 0.005 inch nozzle and a 0.007 inch nozzle. Line 40 was fitted to the data points produced using a 0.005 inch nozzle opening and line 42 was fitted to the data points for a 0.007 inch nozzle opening. Lines 40, 42 were found to be almost collinear.

At a smaller nozzle size (0.005), the pressure increases but the volumetric flow rate decreases, while a larger nozzle size results in an increases in volumetric flow rate but a decrease in pressure. However, as shown in FIG. 3, the cutting performance was generally independent of nozzle diameter (variation in pressure and flow rate) as long as the overall jet power was the same. Thus, holding jet power constant produces a cutting stream that generally has the same effect regardless of the pressure and flow rate.

In one embodiment, the jet power of system 10 is directly measured with pressure and flow rate sensors such as sensors 32 illustrated in FIG. 1. The jet power variables are then fed back to processor 30 which uses these variables to calculate the proper pump settings. System 10 thus provides consistent output power (and cutting depth) regardless of system variations.

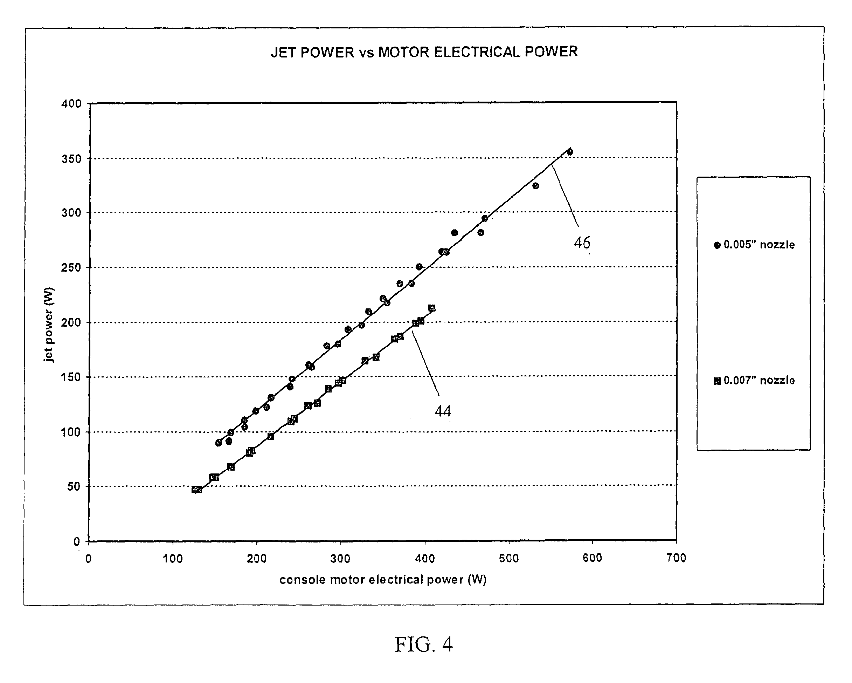

In another embodiment, jet power is controlled indirectly. For example, Applicant has found that the power delivered to the pump motor (e.g., Watts of power) can be controlled to achieve a desired jet power. Directly measuring fluid pressure and flow rate can be complicated, so controlling pump motor power facilitates the control of system 10. FIG. 4 illustrates that using pump power as a controller set point provides an efficient way to control system 10. Applicant measured the jet power produced by system 10 for pump motor power over a range of about 110 Watts to about 600 Watts. One set of measurements were taken with a nozzle diameter of about 0.005 inches and a second set of measurements were taken with a nozzle diameter of about 0.007 inches. Line 44 was fit to the data produced with a 0.007 inch nozzle diameter and line 46 was fit to the data produced with a 0.005 inch nozzle diameter. As shown, the effect of system variations, which caused variations in flow rate and pressure, had a minimal effect on the jet power for a given pump motor power.

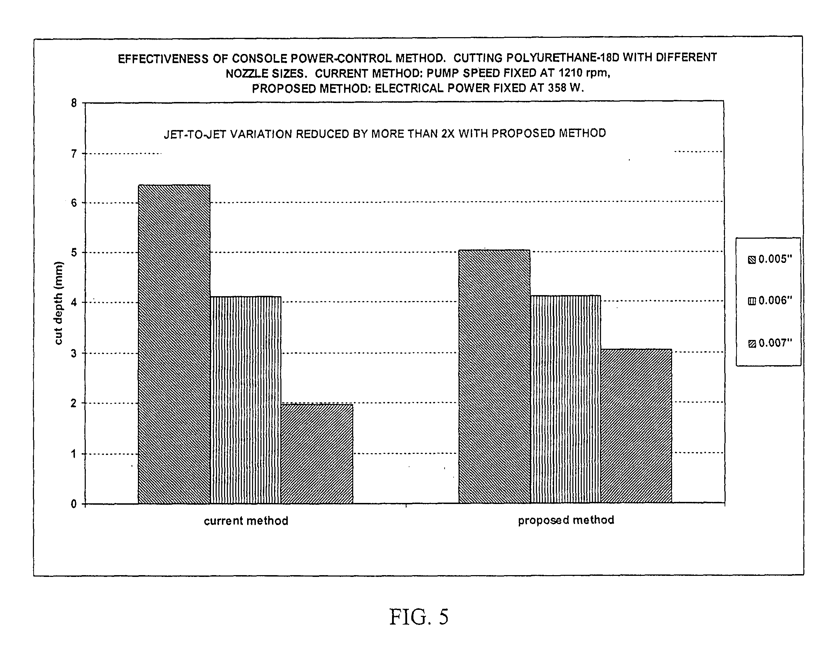

Using pump power to control the fluid cutting jet provides an advantage over conventional fluid jet control systems. In fact, compared to prior art systems in which the fluid stream is controlled by controlling pump speed, Applicant has found that variations in the fluid cutting jet system created less variations in cutting performance when pump power is used as a control variable. For example, FIG. 5 illustrates the cut depth for three different nozzle sizes when pump speed is fixed (bars on the left of the graph) compared to the cut depth for the same three nozzle sizes when pump power is fixed (bars on the right side of the graph). The cutting depth for a system controlled with pump speed varied to a greater degree with changes in nozzle diameter than a system controlled with pump power. Thus, these measurements show that variations in system 10 (e.g., nozzle size) produce less variation in cutting performance (e.g., cutting depth) when pump power is used to control the fluid cutting stream.

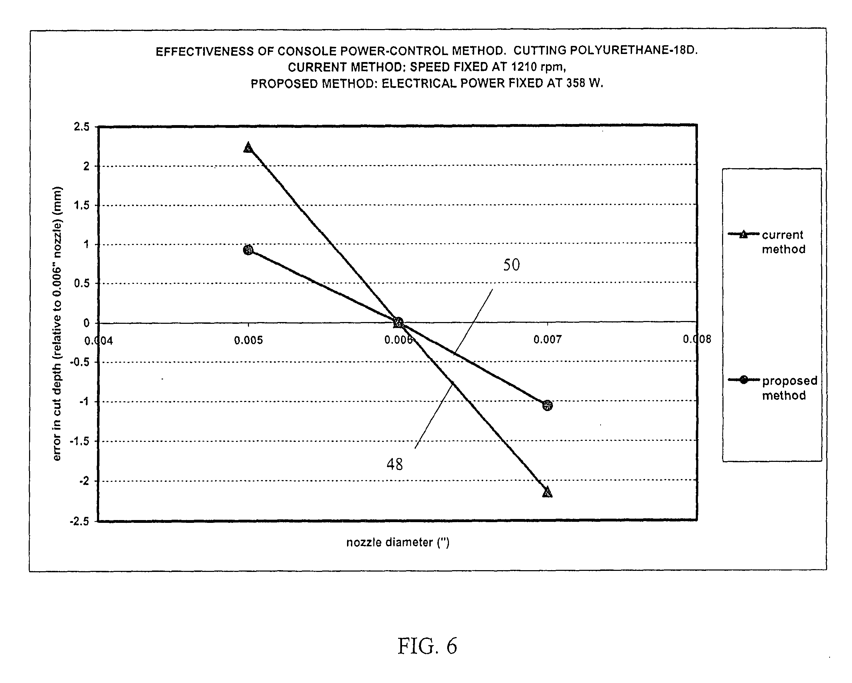

FIG. 6 illustrates this same concept in a linear graph form. The graph shows the variation in cut depth for three nozzle sizes (0.005 inches, 0.006 inches, and 0.007 inches) at constant pump power and at constant pump speed. Line 48 was fit to the data for constant pump speed, while line 50 was fit to the data for constant pump power. Note that line 50 has a much shallower slope indicating that the variation in cut depth for different nozzle diameters is less for constant pump power.

In one embodiment, Applicant therefore controls system 10 by controlling pump power. When a user enters a set point in console 14, processor 30 determines the proper pump power and controls the pump power to achieve the desired cutting performance.

To assist with controlling pump power, a feedback control system can be used. In one embodiment, the processor 30 receives data representative of the pump power to assist with feedback control of the system. In one embodiment, sensors positioned on the pump motor 22 and/or pump assembly 24 can provide processor 30 with feedback input. Such input can be, by way of non-limiting example, the actual power delivered to the pump motor (e.g., watts of power delivered), pump activity (e.g., rpm of the pump motor or speed of the pump piston), flow rate of the cutting fluid, and/or pressure of the fluid exiting pump 12. Based on the data delivered by sensors 32, controller 30 can fine tune the power delivered to pump 12. In another embodiment, other feedback data can be sent to processor 30 to assist with system control. For example, processor 30 can receive data representative of other variable(s) that are related to jet power (e.g., pressure and/or flow rate data).

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

**Claims**

1. A fluid jet cutting system controller, comprising:

   a fluid pump for pressurizing fluid, including a fluid inlet and a fluid outlet, the pump having a pump motor for driving the pump;
   an end effector in fluid communication with the fluid pump and including an orifice for delivering pressurized fluid to tissue; and
   a control console electrically connected to the pump motor and adapted to control the pump motor power level.

2. The system of claim 1, wherein the control console includes a processor adapted to receive input from a user and send an output signal to control the pump motor power level.

3. The system of claim 2, wherein the processor can receive a feedback signal from a sensor and can vary the output signal in response to the feedback signal.

4. The system of claim 3, wherein the sensor is positioned to monitor the pump.

**5.** The system of claim 4, wherein the sensor measures power delivered to the pump.

**6.** The system of claim 3, wherein the sensor is positioned on the end effector.

**7.** The system of claim 3, wherein the sensor measures flow rate.

**8.** The system of claim 3, wherein the sensor measures pressure.

**9.** The system of claim 1, wherein the pump motor is an electric motor and the control console varies the electrical power delivered to the pump.

**10.** The system of claim 1, including a delivery tube for receiving pressurized fluid from the pump and delivering the fluid to the end effector.

**11.** The system of claim 10, wherein the end effector is detachably connected to the delivery tube.

**12.** The system of claim 10, including multiple end effectors.

**13.** The system of claim 1, wherein the end effector includes an exit lumen for the removal of cutting fluid.

**14.** A method of controlling a fluid cutting jet, comprising:

providing a fluid cutting jet system including a fluid pump for pressurizing fluid, the pump having a pump motor for driving the pump, a control console electrically connected to the pump motor and adapted to vary the pump motor power level, and an end effector including a nozzle for delivering pressurized fluid to tissue;
inputting a set point; and
controlling the pump motor power level in response to the set point.

**15.** The method of claim 14, wherein the set point is representative of fluid jet power

**16.** The method of claim 14, wherein the control console includes a processor that receives the set point and an output that sets the pump motor power level.

**17.** The method of claim 14, including the step of providing a feedback signal to the control console from at least one sensor positioned to monitor a system variable of the fluid cutting jet system.

**18.** The method of claim 17, including the step of varying the pump motor power level in response to the feedback signal.

**19.** A fluid jet cutting controller system, comprising:

a fluid pump for pressurizing fluid, including a fluid inlet and a fluid outlet, the pump having a pump motor for driving the pump;
a delivery tube for receiving pressurized fluid from the fluid pump and delivering the fluid to an end effector; and
a control console electrically connected to the pump motor and adapted to control the jet power of a fluid cutting fluid by varying pump parameters.

**20.** The system of claim 19, wherein the control console includes a processor that can receive input from a user and send an output signal to control the pump motor power level.

**21.** The system of claim 20, wherein the processor can receive a feedback signal from a sensor and can vary the output signal in response to the feedback signal.

**22.** The system of claim 21, wherein the sensor measures flow rate.

**23.** The system of claim 21, wherein the sensor measures pressure.

**24.** The system of claim 21, wherein the sensor measures power delivered to the pump.

**25.** The system of claim 19, wherein the pump motor is an electric motor and the control console varies the electrical power delivered to the pump.

**26.** The system of claim 19, including multiple end effectors.

FIG. 1

FIG. 2

JET CUTTING POLYURETHANE-18D
(CUTTING SPEED = 1 mm/s, TARGET DISTANCE = 3 mm)

FIG. 3

EP 1 683 495 A1

FIG. 4

EP 1 683 495 A1

EFFECTIVENESS OF CONSOLE POWER-CONTROL METHOD. CUTTING POLYURETHANE-18D WITH DIFFERENT NOZZLE SIZES. CURRENT METHOD: PUMP SPEED FIXED AT 1210 rpm, PROPOSED METHOD: ELECTRICAL POWER FIXED AT 358 W.

JET-TO-JET VARIATION REDUCED BY MORE THAN 2X WITH PROPOSED METHOD

0.005"
0.006"
0.007"

cut depth (mm)

current method    proposed method

FIG. 5

FIG. 6

**EP 1 683 495 A1**

European Patent
Office

# EUROPEAN SEARCH REPORT

Application Number

EP 06 25 0250

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 505 729 A (RAU ET AL) 9 April 1996 (1996-04-09)<br><br>* the whole document * | 1-3,6, 8-21,23, 25,26 | INV.<br>A61B17/32 |
| X | US 5 259 842 A (PLECHINGER ET AL) 9 November 1993 (1993-11-09)<br>* column 1, line 40 - column 3, line 58 * | 1-3, 8-21,26 | |
| X | US 6 290 670 B1 (PEIN ANDREAS) 18 September 2001 (2001-09-18)<br>* the whole document * | 1-3,14, 19 | |
| X | US 2002/111579 A1 (MOUTAFIS TIMOTHY E ET AL) 15 August 2002 (2002-08-15)<br>* paragraph [0094] - paragraph [0095] * | 1-3,6-26 | |
| X | US 2002/050197 A1 (MOUTAFIS TIMOTHY E ET AL) 2 May 2002 (2002-05-02)<br>* paragraph [0018] * | 1,14,19 | |
| A | EP 0 657 150 A (SENTINEL MEDICAL, INC) 14 June 1995 (1995-06-14)<br>* the whole document * | 1-26 | TECHNICAL FIELDS SEARCHED (IPC)<br><br>A61B |

The present search report has been drawn up for all claims

| Place of search | Date of completion of the search | Examiner |
|---|---|---|
| Munich | 16 May 2006 | Held, G |

EPO FORM 1503 03.82 (P04C01)

14

**ANNEX TO THE EUROPEAN SEARCH REPORT
ON EUROPEAN PATENT APPLICATION NO.**                    EP 06 25 0250

This annex lists the patent family members relating to the patent documents cited in  the above-mentioned European search report.
The members are as contained in the European Patent Office EDP file on
The European Patent Office is in no way liable for these particulars which are merely  given for the purpose of information.

16-05-2006

| Patent document cited in search report | | Publication date | Patent family member(s) | | Publication date |
|---|---|---|---|---|---|
| US 5505729 | A | 09-04-1996 | DE | 9200452 U1 | 04-06-1992 |
| | | | EP | 0555549 A1 | 18-08-1993 |
| | | | JP | 5337125 A | 21-12-1993 |
| US 5259842 | A | 09-11-1993 | DE | 4201992 A1 | 29-07-1993 |
| | | | EP | 0554558 A1 | 11-08-1993 |
| | | | JP | 6063055 A | 08-03-1994 |
| | | | JP | 6098145 B | 07-12-1994 |
| US 6290670 | B1 | 18-09-2001 | AT | 296579 T | 15-06-2005 |
| | | | CA | 2287107 A1 | 26-04-2000 |
| | | | DE | 59812834 D1 | 07-07-2005 |
| | | | DK | 0997105 T3 | 26-09-2005 |
| | | | EP | 0997105 A1 | 03-05-2000 |
| | | | ES | 2244028 T3 | 01-12-2005 |
| | | | HU | 9903849 A2 | 28-06-2000 |
| | | | JP | 2000126222 A | 09-05-2000 |
| | | | KR | 2000029328 A | 25-05-2000 |
| | | | PL | 336202 A1 | 08-05-2000 |
| | | | RU | 2240832 C2 | 27-11-2004 |
| US 2002111579 | A1 | 15-08-2002 | AT | 306222 T | 15-10-2005 |
| | | | AU | 773181 B2 | 20-05-2004 |
| | | | AU | 5028900 A | 05-12-2000 |
| | | | CA | 2373687 A1 | 23-11-2000 |
| | | | DE | 60023136 D1 | 17-11-2005 |
| | | | EP | 1182974 A1 | 06-03-2002 |
| | | | JP | 2002543913 T | 24-12-2002 |
| | | | WO | 0069348 A1 | 23-11-2000 |
| | | | US | 2002177802 A1 | 28-11-2002 |
| | | | US | 6375635 B1 | 23-04-2002 |
| US 2002050197 | A1 | 02-05-2002 | NONE | | |
| EP 0657150 | A | 14-06-1995 | CA | 2111278 A1 | 14-06-1995 |
| | | | US | 5322504 A | 21-06-1994 |

EPO FORM P0459

For more details about this annex : see Official Journal of the European Patent Office, No. 12/82